# EUROPEAN PATENT APPLICATION

(11) **EP 4 653 422 A1**
(43) Date of publication of application: **26.11.2025**
(21) Application number: 24913477.6
(22) Date of filing: 27.11.2024
(51) Int. Cl.: C07D 207/267

(54) **SYSTEM FOR PURIFYING N-METHYL-2-PYRROLIDONE, AND METHOD THEREFOR**

(30) Priority: 27.12.2023 KR 20230192358
(71) Applicant: Solis Co., Ltd., Cheonan-si, Chungcheongnam-do 31036 (KR)
(72) Inventor: LEE, Jongpil, Hwaseong-si Gyeonggi-do 18443 (KR); JEOUNG, Daesuk, Hwaseong-si Gyeonggi-do 18508 (KR)
(74) Representative: Jung, Minkyu
(86) International application number: PCT/KR2024/019042
(87) International publication number: WO 2025/143584

(57) **Abstract**

The present invention relates to a system for purifying N-methyl-2-pyrrolidone, and a method therefor, and, to an NMP purification system comprising: a first module unit including a booster fan unit for collecting waste NMP gas, and a waste NMP gas adsorption unit for adsorbing the waste NMP gas and converting same into a waste NMP solution; a second module unit which is adjacent to the first module unit, and which includes a DI water supply unit for supplying DI water used for the waste NMP gas adsorption unit; a third module unit which is adjacent to the second module unit, and which includes a first purification unit for purifying, through permeation and evaporation, the waste NMP solution supplied from the first module unit; a fourth module unit which is adjacent to the third module unit, and which includes a second purification unit for purifying, through distillation, the purified NMP solution supplied from the third module unit, and a third purification unit for purifying, through distillation, the NMP solution purified in the second purification unit; and a fifth module unit which is adjacent to the fourth module unit, and which includes a fourth purification unit for purifying, through vaporization, the purified NMP solution supplied from the fourth module unit.

## Description

### Technical Field

The present invention relates to an N-methyl-2-pyrrolidone purification system and a method therefor, wherein energy efficiency is high, it is easy to install and manage the system, and it is possible to stably perform N-methyl-2-pyrrolidone purification.

### Background Art

Recently, with the popularization of electric vehicles, the secondary battery industry is growing rapidly. Secondary batteries are composed of positive electrode materials, negative electrode materials, and electrolytes, and are manufactured through a packaging process. A positive electrode material, which is an active material used in a positive electrode electrode, is an electrode that is reduced during the discharge of a secondary battery, and the negative electrode material, which is an active material used in a negative electrode electrode, is an electrode that releases electrons to a conductor through an oxidation reaction during the discharge of a secondary battery.

A binder is required to manufacture positve electrode materials. A binder is composed of a combination of polyvinylidene fluoride (PVDF) and N-methyl-2-pyrrolidone (NMP), and a lot of NMP is required for the binder for a positive electrode material. A binder using PVDF and NMP has excellent adhesive strength, facilitates the dispersion of conductive materials, and can maintain a stable state even during oxidation/reduction reactions. Pure NMP contained in a binder is mostly volatilized into gas by heat and pressure during a positive electrode material coating process in a secondary battery manufacturing process, and the volatilized NMP can be captured and recycled. NMP regeneration methods can be largely divided into three types depending on the capture method of used NMP gas, the purification method of captured NMP, and the location of a used NMP collector and NMP purifier.

The classification according to the capture method of used NMP gas includes a dry capture method and a wet method, and the purification method of the captured NMP includes a distillation method, rectification method, stripping vessel method, and adsorption method; the classification according to the location of a collector and purifier is a method of regenerating, using an external waste NMP purification system, waste NMP liquid that has been state-converted by adsorbing pure water to used NMP gas through capture and on-site where the collector and purifier are connected.

In general, about 1,000 tons of waste NMP are generated per 1 GWh of battery capacity in a secondary battery manufacturing process. Looking at the component of waste NMP, the waste NMP is composed of about 72% NMP, about 20% moisture, about 7% nitride compounds, and about 1% polymer and other impurities such as particles. Therefore, an economical and stable method for regenerating waste NMP is needed.

### Detailed Description of the Invention

### Technical Problem

The present invention relates to an NMP purification system and a method therefor, wherein energy efficiency is high and mass regeneration is possible.

In addition, the present invention relates to an NMP purification system and a method therefor, wherein it is possible to reduce the installation cost of an NMP purification system and shorten the installation period.

In addition, the present invention relates to an NMP purification system and a method therefor, wherein it is possible to efficiently manage an NMP purification process and prevent safety accidents.

### Technical Solution

The present invention relates to an NMP purification system, including: a first module unit including a booster fan unit for collecting waste NMP gas, and a waste NMP gas adsorption unit for adsorbing the waste NMP gas and converting the same into a waste NMP solution; a second module unit being adjacent to the first module unit, and including a DI water supply unit for supplying DI water used for the waste NMP gas adsorption unit; a third module unit being adjacent to the second module unit, and including a first purification unit for purifying, through permeation and evaporation, the waste NMP solution supplied from the first module unit; a fourth module unit being adjacent to the third module unit, and including a second purification unit for purifying, through distillation, the purified NMP solution supplied from the third module unit, and a third purification unit for purifying, through distillation, the NMP solution purified in the second purification unit; and a fifth module unit being adjacent to the fourth module unit, and including a fourth purification unit for purifying, through vaporization, the purified NMP solution supplied from the fourth module unit.

Here, the NMP purification system may further include a first module connection unit for connecting neighboring modules between the first to fifth module units, wherein the first module connection unit allows a module fixing unit provided at a frame of the outer surface of each of the first to fifth module units to be aligned with a module fixing plate, adds a fixing pad, and then connects them through a bolt.

In addition, each of the first to fifth module units may include: a fixing unit for fixing a device for NMP purification located inside each module; and a ground connection unit under each module unit and for installing each module on the ground, wherein the ground connection unit is installed on the ground and connected to a second module connection unit for installing and fixing each module unit on the ground.

In addition, the third module unit may include a first inspection unit that inspects, through spectrometry, the state of the NMP solution purified in the first purification unit.

In addition, the first inspection unit may inspect the content of moisture contained in purified NMP, and the first purification unit may re-purify the purified NMP if the content of moisture contained in the purified NMP exceeds a preset moisture content.

In addition, the fifth module unit may include a second inspection unit that collects a sample of the NMP solution and examines the same through component analysis method to analyze the state of the NMP solution purified in the fourth purification unit.

In addition, the second inspection unit may include: a sample NMP storage unit for storing a predetermined amount of sample NMP; a sample NMP input unit for putting, into the sample NMP storage unit, a predetermined amount of sample NMP supplied from the outside; a sample NMP transfer unit for transferring the sample NMP from the sample NMP storage unit to a sample NMP container; a sample NMP inspection unit for analyzing the components of the sample NMP stored in the sample NMP container; and a sample NMP container storage unit for storing the sample NMP container.

In addition, NMP purified in at least one of the second to fourth purification units may be re-purified by using at least one of the content of moisture contained in NMP purified in the fourth purification unit or the purity of purified NMP.

In addition, the second and third purification units may purify, by using a distillation tower, a purified NMP solution supplied from the third module unit, wherein the distillation tower used in the second purification unit may include vaporized water located at the upper part and purified NMP located at the lower part, and the distillation tower used in the third purification unit may include vaporized NMP located at the upper part and impurities located at the lower part.

In addition, the fourth purification unit may allow moisture to evaporate as a purified NMP solution supplied from the fourth module unit passes through a passage of a predetermined length.

### Advantageous Effects

The present invention applies an automated method to an NMP purification process, enabling efficient management through real-time monitoring, so that productivity and energy efficiency for NMP purification are high, enabling large-scale NMP regeneration. In addition, according to the present invention, it is possible to configure an NMP purification system in a modular manner, thereby reducing on-site installation costs and shortening the installation period.

### Brief Description of the Drawings

FIG. 1 is a view showing the main configuration of an NMP purification system according to an embodiment of the present invention.
FIG. 2 is a view showing the main configuration of each module unit constituting an NMP purification system according to an embodiment of the present invention.
FIG. 3 is a view showing the configuration of a second inspection unit according to an embodiment of the present invention.
FIG. 4 is a flowchart for explaining an operation of an NMP purification system according to an embodiment of the present invention.

### Mode for Carrying Out the Invention

Hereinafter, embodiments of the present invention will be described in detail with drawings. In describing the present invention, if it is determined that a detailed description of a related known function or configuration may unnecessarily obscure the gist of the present invention, the detailed description will be omitted.

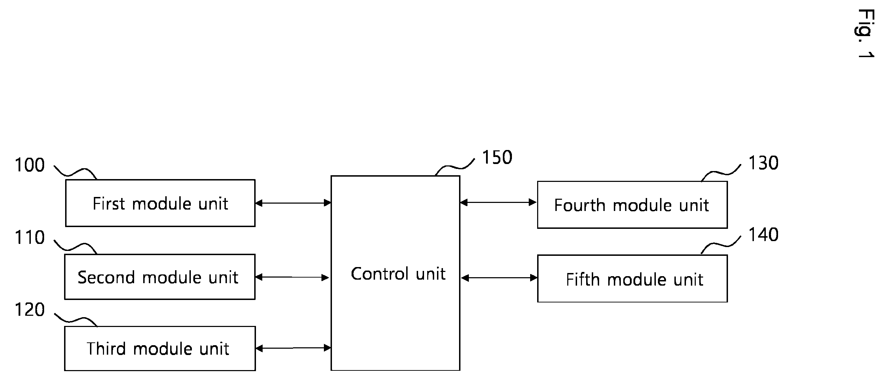

FIG. 1 is a view showing the main configuration of an NMP purification system according to an embodiment of the present invention.

An NMP purification system of the present invention is composed of first to fifth module units (100, 110, 120, 130, 140) and a control unit (150), and each module unit is equipped with a device for NMP purification. Each module unit is manufactured in advance and transferred to a site where the NMP purification system is to be installed, and at the site, each module unit may be connected to install the NMP purification system. For example, the first to fifth module units (100, 110, 120, 130, 140) are configured to be transferable in a container size, and each module unit is manufactured so that each configuration included in each module unit is connected to each other and operable internally, and at the site where the NMP purification system is installed, connection units between respective module units may be connected and installed, like a piping connection.

Each of the first to fifth module units (100, 110, 120, 130, 140) is composed of at least one block, wherein each block is composed of an outer surface composed of multiple frames and a frame for connecting each block to the other, and an H beam may be used for the frame. In addition, each module unit includes a fixing unit for fixing a device equipped for NMP purification inside, and the fixing unit connects a frame equipped on the outer surface of each module unit and a support beam for supporting a device equipped inside each module unit. In addition, a first module connection unit for connecting neighboring module units is provided between the module units, and the first module connection unit aligns a module fixing unit equipped on the frame on the outer surface of each module unit with a module fixing plate, adds a fixing pad, and then connects them through a bolt. This is to minimize damage to other modules when an abnormality occurs in a given module, such as vibration or weakening of the support of a given module on the ground.

Each module unit is installed and fixed on the ground by connecting a ground connection unit provided at the lower part of each module unit and a second module connection unit installed on the ground. The second module connection unit includes a ground-embedded unit and a connection unit, wherein after the ground-embedded unit is embedded in the ground and firmly fixed, the floor-side construction is performed so that floor layer cement is completely cured and the connection unit is exposed above the floor layer. When the ground connection unit provided in each module unit and the connection unit provided in the second module connection unit are aligned and connected through a bolt after adding a fixing pad, each module unit is installed on the ground.

The control unit (150) entirely controls the present invention, for example, by controlling a process for refining waste NMP into high-purity purified NMP.

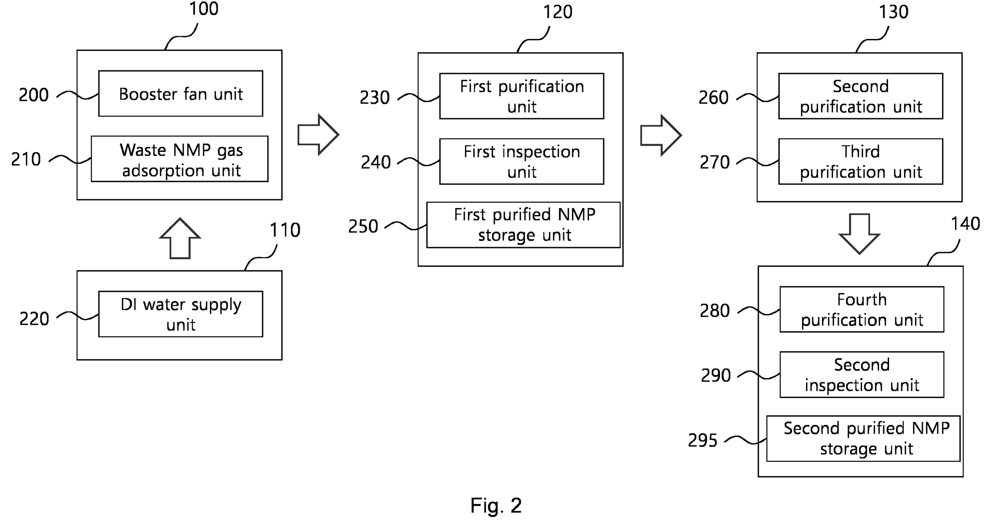

FIG. 2 is a view showing the main configuration of each module unit constituting an NMP purification system according to an embodiment of the present invention.

Referring to FIG. 2, the detailed configuration of the first to fifth module units (100, 110, 120, 130, 140) of the present invention is examined, wherein the first module unit (100) is equipped with a booster fan unit (200) and a waste NMP gas adsorption unit (210), the second module unit (110) is equipped with a DI water supply unit (220), and the third module unit (120) is equipped with a first purification unit (230), a first inspection unit (240), and a first purified NMP storage unit (250). In addition, the fourth module unit (130) is equipped with a second purification unit (260) and a third purification unit (270), and the fifth module unit (140) is equipped with a fourth purification unit (280), a second inspection unit (290), and a second purified NMP storage unit (295). In FIG. 2, each module unit is illustrated with its main configurations, and may further include various configurations in addition to the illustrated configurations. For example, each module unit may be suitably provided with a storage unit for storing waste NMP, a storage unit for storing purified NMP, a sensor unit for detecting the status of devices in each module, and a pipe, wire, pump, valve, container, or the like for transferring waste NMP or purified NMP.

The booster fan unit (200) captures, using a forced exhaust method, waste NMP gas generated during a positive electrode material coding and drying step in a secondary battery production process, and transfers the same to the waste NMP gas adsorption unit (210).

The waste NMP gas adsorption unit (210) converts waste NMP gas into waste NMP liquid containing a large amount of pure distilled water by adsorption of pure distilled water to the waste NMP gas. For example, pure distilled water may be used as deionized water (DI water), and waste NMP liquid may contain about 20 to 30% DI water. The liquefied waste NMP is transferred to a waste NMP storage unit (not shown). At this time, the waste NMP storage unit (not shown) may include a filter for removing various particles such as metal particles and organic particles contained in the waste NMP liquid, wherein the waste NMP liquid stored in the waste NMP storage unit (not shown) may have most of the particles filtered out by the filter, and the main configuration of the waste NMP may be composed of pure distilled water and NMP. The waste NMP liquid from which the particles are removed in the waste NMP storage unit (not shown) undergoes a waste NMP purification process in the first to fourth purification units (230, 280).

The DI water supply unit (220) supplies DI water to the waste NMP gas adsorption unit (210), and may produce and supply DI water.

The first purification unit (230) separates pure distilled water and NMP from the waste NMP liquid, and for example, the first purification unit (230) may use a pervaporation method. A separation membrane used in the pervaporation method is a GET membrane, which is a structure in which PVA of about 0.2 micrometers is grafted onto a porous PAN 100 micrometers support, and a support layer made of polyester nonwoven fabric of about 100 micrometers is supported on the outside. This pervaporation method is a physical regeneration method with high energy efficiency, so it is possible to remove a certain amount of moisture before purification in the second to fourth purification units (260, 280), thereby shortening the distillation time and enhancing energy efficiency.

The NMP purified in the first purification unit (230) is stored in the first purified NMP storage unit (250), and the first inspection unit (240) inspects the components contained in the purified NMP stored in the first purified NMP storage unit (250). For example, a portion of a transfer pipe that introduces or discharges the purified NMP to the first purified NMP storage unit (250) is configured as a transparent pipe, and infrared light or other light is irradiated on the transparent pipe to inspect, in real time, the components of the purified NMP, such as the content of moisture contained in the purified NMP. At this time, the first inspection unit (240) may use the Fourier Transform Near Infrared (FT-NIR) method. In addition, a flow sensor is provided in a pipe for transferring the purified NMP to the first purified NMP storage unit (250), so that the flow rate of the purified NMP transferred through the pipe can be measured, and the amount of the purified NMP stored in the first purified NMP storage unit (250) can be measured through a level sensor provided in the first purified NMP storage unit (250).

The second and third purification units (260, 270) re-purify the NMP purified in the first purification unit (230) using a distillation method, and for example, the second and third purification units (260, 270) may use a distillation tower. Looking at the purification process in the second purification unit (260), the NMP purified in the first purification unit (230) is a mixture of water and NMP, so that it can be divided into low-boiling-point components and high-boiling-point components. In the second purification unit (260), the low-boiling-point components are vaporized first, so that the vaporized water is located at the upper part of the second purification unit (260), and the purified NMP is located at the lower part of the second purification unit (260). The vaporized water at the upper part of the second purification unit (260) is cooled and then transferred to an external tank for processing.

The purified NMP at the lower part of the second purification unit (260) is transferred to the third purification unit (270), and also in the third purification unit (270), the low-boiling-point components are vaporized first, so that the vaporized NMP is located at the upper part of the third purification unit (270), and the impurities are located at the lower part of the third purification unit (270). The purified NMP located at the upper part of the third purification unit (270) is cooled and then transferred to the fourth purification unit (280), and the impurities located at the lower part of the third purification unit (270) are transferred to a waste liquid tank (not shown) for processing.

The purified NMP that has undergone the purification process in the second and third purification units (260, 270) is transferred to the fourth purification unit (280). The fourth purification unit (280) performs the purification process using a vaporization method, for example, by allowing the purified NMP to pass through a passage of a predetermined length so that moisture is vaporized. The purified NMP that has undergone the purification process in the fourth purification unit (280) is stored in the second purified NMP storage unit (295), and the second inspection unit (290) inspects the components of the purified NMP stored in the second purified NMP storage unit (295).

Meanwhile, according to the present invention, it is possible to collect data generated in all processes for purifying waste NMP in a gaseous state into high-purity purified NMP using various sensors. A variety of data such as the temperature, pressure, and flow rate is collected in real time and transmitted to the control unit (150), and a manager can monitor the purification process in real time. For example, the temperature, pressure, storage volume, purity of the stored liquid, and PH of the first and second purified NMP storage units (250, 295) may be measured in real time, and the flow rate and temperature may be measured in real time using sensors mounted on pipes. Such data may be compared with preset normal state data to check for abnormalities, and if abnormalities are found, the quality of the purified NMP can be managed or safety accidents can be prevented in advance by modifying the conditions of the purification process or stopping the purification process as discussed above.

In addition, the control unit (150) may learn using deep learning using artificial neural networks such as Convolution Neural Network (CNN), Deep Neural Network (DNN), and Recurrent Neural Network (RNN), so that it is possible to learn various data to set the optimal state, and it is possible to detect the presence/absence of abnormalities in each process in advance. In addition, it is possible to present optimal process conditions to a manager and notify the manager of process procedures that require changes in process conditions in multiple process procedures to improve the quality of the purified NMP. For example, the control unit (150) may learn the data generated in each process and the data on the purified NMP to derive the corresponding relationship between the data in each process and the purified NMP, and if the quality of the purified NMP does not meet the standard, it is possible to determine which process requires modification, and suggest to the manager the modification of the process conditions in the given process. To this end, the control unit (150) may learn in advance the corresponding relationship between the data generated in the first purification unit (230) and the first purified NMP storage unit (250), and the corresponding relationship between the data generated in the second to fourth purification units (260, 270, 280) and the second purified NMP storage unit (295). In addition, the learned data may be updated, so that the update can be made to reflect modifications to the process conditions that occur during the purification process. In addition, the control unit (150) may connect to the outside through a communication network to collect/analyze information on new technologies or new process conditions related to purification of waste NMP, and provide related information to the manager, and based on the collected/analyzed data, may suggest to a manager to change certain process conditions in the waste NMP processes of the present invention.

In addition, if the content of moisture contained in the purified NMP stored in the first purified NMP storage unit (250) exceeds a preset moisture content, the purified NMP stored in the first purified NMP storage unit (250) may be transferred to the first purification unit (230) to be re-purified. In addition, if the flow rate of the purified NMP through a pipe exceeds a preset amount, the flow rate may be reduced, and if the amount of the purified NMP stored in the first purified NMP storage unit (250) exceeds a preset amount, the purification in the first purification unit (230) may be stopped or the purified NMP may be controlled not to be discharged from the first purification unit (230) to the first purified NMP storage unit (250).

In addition, if the content of moisture contained in the purified NMP stored in the second purified NMP storage unit (295) exceeds a preset moisture content, the process conditions applied in the purification process in the second to fourth purification units (260, 280), such as temperature control or purification time control, may be changed, or the purification process in the second to fourth purification units (260, 290) may be performed again for the purified NMP stored in the second purified NMP storage unit (295) to purify the NMP. For example, if the content of moisture contained in the purified NMP stored in the second purified NMP storage unit (295) is greater than or equal to a first target value, the purified NMP stored in the second purified NMP storage unit (295) may be transferred to perform the purification process in the second to fourth purification units (260, 290) again, and if the content of moisture contained in the purified NMP stored in the second purified NMP storage unit (295) is less than the first target value but greater than or equal to a second target value, the purification process in the third and fourth purification units (270, 280) may be performed again, and if the content of moisture contained in the purified NMP stored in the second purified NMP storage unit (295) is less than the second target value but greater than or equal to a third target value, the purification process in the fourth purification unit (280) may be performed again.

In addition, if the purity of the purified NMP stored in the second purified NMP storage unit (295) is less than a preset value, the purification process in the second to fourth purification units (260, 290) may be performed again to purify the NMP. For example, if the purity of the purified NMP stored in the second purified NMP storage unit (295) is less than the first target value, the purified NMP stored in the second purified NMP storage unit (295) may be transferred to perform the purification process in the second to fourth purification units (260, 280) again, and if the purity of the purified NMP stored in the second purified NMP storage unit (295) exceeds the first target value but is less than the second target value, the purification process in the third and fourth purification units (270, 280) may be performed again, and if the purity of the purified NMP stored in the second purified NMP storage unit (295) exceeds the second target value but is less than the third target value, the purification process in the fourth purification unit (280) may be performed again.

The control unit (150) may control the purification process by using either the content of moisture contained in the purified NMP or the purity of the purified NMP, or both.

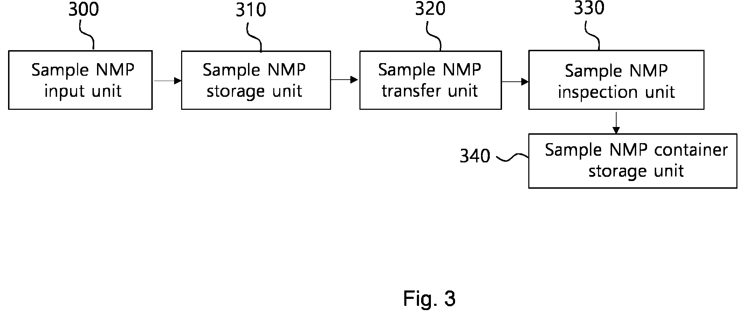

FIG. 3 is a view showing the configuration of a second inspection unit (290) according to an embodiment of the present invention.

Referring to FIG. 3, the second inspection unit (290) includes a sample NMP input unit (300), a sample NMP storage unit (310), a sample NMP transfer unit (320), a sample NMP inspection unit (330), and a sample NMP container storage unit (340).

The sample NMP input unit (300) is located between the second purified NMP storage unit (295) and the sample NMP storage unit (310), and puts, into the sample NMP storage unit (310), a predetermined amount of sample NMP from among the purified NMP stored in the second purified NMP storage unit (295). To this end, the sample NMP input unit (300) includes an automatic valve such as a solenoid valve, and is opened and closed under the control of the control unit (150). For example, the control unit (150) may control the sample NMP input unit (300) to be closed when the level of sample NMP stored in the sample NMP storage unit (310) exceeds a preset level value and to be opened when the level is less than or equal to the preset level value.

The sample NMP storage unit (310) stores the sample NMP supplied from the second purified NMP storage unit (295), and the stored sample NMP is transferred to and stored in a sample NMP container.

The sample NMP transfer unit (320) is located between the sample NMP storage unit (310) and the sample NMP container and transfers the sample NMP stored in the sample NMP storage unit (310) to the sample NMP container. For example, the sample NMP transfer unit (320) may include a nozzle and a cylinder including a piston, and a fine nozzle may be used as the nozzle. The sample NMP provided from the sample NMP storage unit (310) is transferred through the cylinder, and the cylinder may be pumped by the movement of the piston. The fine nozzle is located between the cylinder and an NMP container so that the sample NMP output from the cylinder is transferred to the NMP container through the fine nozzle.

The sample NMP inspection unit (330) analyzes the components of the NMP using the sample NMP stored in the sample NMP container and transmits the analysis data to the control unit (150). For example, the sample NMP inspection unit (330) may inspect the content of moisture, particles, etc. contained in the sample NMP.

The sample NMP container storage unit (340) stores the sample NMP container that has been inspected by the sample NMP inspection unit (330). The sample NMP container is stored in a predetermined amount in the sample NMP container storage unit (340) and then discharged to the outside at predetermined time intervals.

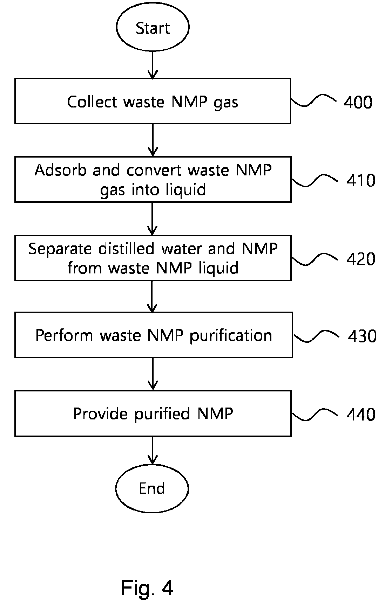

FIG. 4 is a flowchart for explaining an operation of an NMP purification system according to an embodiment of the present invention.

Referring to FIG. 4, when waste NMP gas generated in a secondary battery production process is captured in the booster fan unit (200) and provided to the waste NMP gas adsorption unit (210) (400), the waste NMP gas adsorption unit (210) adsorbs the waste NMP gas and converts it into liquid (410). Thereafter, the first purification unit (230) separates distilled water and waste NMP from the liquid waste NMP (420), and the waste NMP from which the distilled water has been separated is purified in the second to fourth purification units (260, 270, 280) (430). The purified NMP is provided so as to be reused in a secondary battery production process (440), and the purified NMP may be exported to the outside and used for other purposes.

Meanwhile, although the detailed description of the present invention has described specific embodiments, it is obvious that various modifications are possible within the scope of the present invention.

Therefore, the scope of the present invention is not limited to the described embodiments, but should be determined by the scope of the claims described below as well as the equivalents of the scope of these claims.

## Claims

1. An NMP purification system, comprising:
a first module unit comprising a booster fan unit for collecting waste NMP gas, and a waste NMP gas adsorption unit for adsorbing the waste NMP gas and converting the same into a waste NMP solution;
a second module unit being adjacent to the first module unit, and comprising a DI water supply unit for supplying DI water used for the waste NMP gas adsorption unit;
a third module unit being adjacent to the second module unit, and comprising a first purification unit for purifying, through permeation and evaporation, the waste NMP solution supplied from the first module unit;
a fourth module unit being adjacent to the third module unit, and comprising a second purification unit for purifying, through distillation, the purified NMP solution supplied from the third module unit, and a third purification unit for purifying, through distillation, the NMP solution purified in the second purification unit; and
a fifth module unit being adjacent to the fourth module unit, and comprising a fourth purification unit for purifying, through vaporization, the purified NMP solution supplied from the fourth module unit.

2. The NMP purification system of claim 1, further comprising a first module connection unit for connecting neighboring modules between the first to fifth module units, wherein the first module connection unit allows a module fixing unit provided at a frame of the outer surface of each of the first to fifth module units to be aligned with a module fixing plate, adds a fixing pad, and then connects them through a bolt.

3. The NMP purification system of claim 1, wherein each of the first to fifth module units comprises:
a fixing unit for fixing a device for NMP purification located inside each module; and
a ground connection unit under each module unit and for installing each module on the ground,
wherein the ground connection unit is installed on the ground and connected to a second module connection unit for installing and fixing each module unit on the ground.

4. The NMP purification system of claim 1, wherein the third module unit comprises a first inspection unit that inspects, through spectrometry, the state of the NMP solution purified in the first purification unit.

5. The NMP purification system of claim 4, wherein the first inspection unit inspects the content of moisture contained in purified NMP, and the first purification unit re-purifies the purified NMP if the content of moisture contained in the purified NMP exceeds a preset moisture content.

6. The NMP purification system of claim 1, wherein the fifth module unit comprises a second inspection unit that collects a sample of the NMP solution and examines the same through component analysis method to analyze the state of the NMP solution purified in the fourth purification unit.

7. The NMP purification system of claim 6, wherein the second inspection unit comprises:
a sample NMP storage unit for storing a predetermined amount of sample NMP;
a sample NMP input unit for putting, into the sample NMP storage unit, a predetermined amount of sample NMP supplied from the outside;
a sample NMP transfer unit for transferring the sample NMP from the sample NMP storage unit to a sample NMP container;
a sample NMP inspection unit for analyzing the components of the sample NMP stored in the sample NMP container; and
a sample NMP container storage unit for storing the sample NMP container.

8. The NMP purification system of claim 1, wherein NMP purified in at least one of the second to fourth purification units is re-purified by using at least one of the content of moisture contained in NMP purified in the fourth purification unit or the purity of purified NMP.

9. The NMP purification system of claim 1, wherein the second and third purification units purify, by using a distillation tower, a purified NMP solution supplied from the third module unit, wherein the distillation tower used in the second purification unit includes vaporized water located at the upper part and purified NMP located at the lower part, and the distillation tower used in the third purification unit includes vaporized NMP located at the upper part and impurities located at the lower part.

10. The NMP purification system of claim 1, wherein the fourth purification unit allows moisture to evaporate as a purified NMP solution supplied from the fourth module unit passes through a passage of a predetermined length.
